# DEMANDE DE BREVET EUROPEEN

(11) **EP 0 714 655 A1**
(43) Date de publication de la demande: **05.06.1996**
(21) Numéro de dépôt: 95402597.9
(22) Date de dépôt: 17.11.1995
(51) Int. Cl.: A61K 7/32, A61K 7/36

(54) **Composition cosmétique et procédé pour lutter contre les odeurs corporelles désagréables produites par la transpiration**

(30) Priorité: 18.11.1994 FR 9413856
(71) Demandeur: LABORATOIRE DE BIOLOGIE VEGETALE YVES ROCHER, F-56201 La Gacilly (FR)
(72) Inventeur: Holtzinger, Gérard, F-93200 Saint-Denis (FR)
(74) Mandataire: Le Guen, Gérard

(57) **Abrégé**

L'invention a pour objet un procédé pour lutter contre les odeurs corporelles désagréables émises au cours du processus de transpiration ainsi qu'une composition cosmétique comprenant :
(A) - un composé consistant en un inhibiteur enzymatique de la β-glucuronidase et de l'arylsulfatase;
(B) - un composé permettant d'élever le pH cutané au niveau de zones de transpiration à pH compris entre 7 et 8, de préférence entre 7 et 7,5;
(C) - un composé diminuant la biodisponibilité de l'eau, ledit composé étant notamment choisi parmi les substances présentes dans la transpiration à l'état naturel; et
- un véhicule à usage cosmétique.

## Description

La présente invention concerne une composition ainsi qu'un procédé pour inhiber la formation d'odeurs corporelles désagréables émises pendant le processus de transpiration.

Le processus de transpiration est dû aux glandes sudoripares :
- les glandes sudoripares eccrines que l'on trouve sur l'ensemble du corps : leur densité (140 à 350 par cm²) dépend de la région corporelle et est adaptée au climat (les populations des régions chaudes ont plus de glandes eccrines que celles des régions froides). Ces glandes sont particulièrement abondantes sur le front, la tête, les aisselles, les paumes et les plantes de pieds;
- les glandes sudoripares apocrines sont relativement peu nombreuses et ne se développent qu'à la puberté. On ne les trouve que dans les zones poilues : aisselles, organes génitaux et anus principalement. Ces glandes apocrines ressemblent aux eccrines, mais le canal excréteur s'ouvre dans un follicule pilo-sébacé près de la surface cutanée au-dessus de l'abouchement du canal sébacé.

Au cours de son émission, la composition de la sueur varie en passant d'une sueur dite primitive à la sueur définitive. La sueur est composée principalement :
. d'eau à environ 99%
. d'acides aminés basiques essentiellement, les acides aminés acides étant trois fois moins abondants
. des substances azotées dont l'urée
. des acides organiques (citrique, formique, lactique, urocanique)
. des vitamines (B6, B5, H)
. de l'acide paraaminobenzoïque, de l'inositol
. des sels minéraux : Na, K, Ca, Mg, Fe, chlorures, sulfates et phosphates
. des enzymes qui jouent un rôle important dans la formation des odeurs corporelles
. du glycogène et du glucose.

La sudation dépend principalement de 3 facteurs :
- thermique;
- psychique : un stress induit une sudation essentiellement localisée au front, paumes, aisselles, plantes de pieds; c'est la "sueur froide";
- pharmacologique par l'acétylcholine ou l'adrénaline.

Les aisselles et les zones génitales sont un lieu idéal pour la croissance bactérienne et la sueur y contribue de par :
. une teneur en eau élevée,
. un niveau nutritif satisfaisant,
. une température favorable.

Ceci explique un niveau élevé de bactéries : environ 10⁶ par cm² de peau.

Les microorganismes trouvés sur la peau des aisselles sont représentatifs de la flore indigène de la peau : 70% de la flore est représentée par les corynébactéries.

La sueur est un mélange de sueurs apocrine et eccrine et de sécrétions sébacées et contient, à faible dose, des stéroïdes. Lorsque ce mélange arrive sur une peau propre, il est inodore, mais sous l'action de certaines enzymes d'origine bactérienne, les stéroïdes vont être dégradés en composés fortement odorants.

Ce mécanisme a été particulièrement étudié par : C. FROEBE, A. SIMONE, A. CHARIG et E. EIGEN : Axillary malodor production : a new mechanism - J. Soc. Cosmet. Chem. 41, 173-185 (May - June 1990).

Un système a été proposé pour inhiber la formation de stéroïdes volatils, odorants par : C. CHARIG, C. FROEBE, A. SIMONE et E. EIGEN : Inhibitor of odor-producing axillary bacterial exoenzymes J. Soc. Cosmet. Chem. 41, 133-145 (May - June 1991).

Cet article propose l'utilisation d'un sel organique de zinc (le glycinate de zinc) agissant comme inhibiteur de la ß-glucuronidase et de l'aryl-sulfatase pour combattre les mauvaises odeurs produites par la transpiration.

De manière générale, les produits cosmétiques utilisés pour combattre les odeurs désagréables liées à la transpiration agissent à l'un ou l'autre des trois niveaux suivants :
- sur les glandes sudoripares en bloquant le processus de transpiration ou en agissant comme régulateurs de la transpiration;
- sur la dégradation de la sueur en agissant comme bactéricides enzymatiques ou non enzymatiques;
- sur l'émission d'odeurs liées à la décomposition de la sueur par les enzymes bactériennes en agissant, comme parfums ou absorbeurs d'odeurs.

La présente invention propose un système global visant à agir simultanément à différents stades du processus de transpiration afin de diminuer de façon efficace les odeurs désagréables générées au cours du processus de transpiration dans sa totalité (depuis l'excrétion de la sueur par les glandes sudoripares jusqu'à l'émission d'odeurs désagréables liée aux processus enzymatiques de dégradation de la sueur).

A ce jour aucun procédé, ni aucune composition ayant une action globale sur plusieurs stades du processus de transpiration ne sont connus.

Les seuls composés ayant une action multiple sont les agents antitranspirants qui possèdent également une action bactéricide.

La présente invention se propose donc de remédier aux problèmes des odeurs désagréables liées à la transpiration notamment au niveau des aisselles en agissant à la fois :

1.- sur la peau :
. en augmentant légèrement le pH cutané au niveau des zones de transpiration.

Le pH de la sueur est compris entre 4 et 6,8. Grâce à l'invention on élève ce pH à 7-8, de préférence 7-7,5 notamment grâce à un système tampon bien toléré par la peau.

L'élévation à pH légèrement alcalin du pH cutané a pour effet de prévenir la formation des acides gras volatils dont l'émission est l'une des causes principales des odeurs désagréables.

Avantageusement selon l'invention,cette augmentation légère du pH est réalisée grâce à l'apport de substances ayant un effet tampon déjà présentes dans la sueur à l'état naturel, en particulier des aminoacides basiques.
. en diminuant la biodisponibilité de l'eau au niveau des zones de transpiration, ce qui a pour effet de freiner le développement des bactéries donc de limiter la dégradation de la sueur et de favoriser l'inhibition enzymatique des enzymes précitées à l'origine de la dégradation des stéroïdes contenus dans la sueur;
. de façon facultative en stabilisant le développement bactérien de la sueur, de préférence à l'aide de composés présents dans la sueur à l'état naturel.

2. - par inhibition directe des enzymes secrétées par les bactéries de la peau, notamment les corynebactéries : la β-glucuronidase et l'aryl sulfatase.

Cette inhibition est obtenue en utilisant un composé inhibant simultanément l'action de ces deux enzymes.

Les travaux des inventeurs les ont amenés à découvrir qu'un inhibiteur puissant de ces deux enzymes était constitué par un sel minéral de zinc, en solution aqueuse, notamment le sulfate de zinc.

3. - de manière facultative par blocage des acides gras volatils qui constituent avec les stéroïdes volatils le second groupe de composants de la sueur jugé responsable des odeurs désagréables.

Cette action peut être obtenue en ajoutant au niveau des zones de transpiration des composés aptes à piéger les acides gras volatils, par exemple en réalisant une liaison chimique entre deux chaînes d'acides gras courtes.

4. - de manière facultative par addition d'un parfum adapté.

Dans l'odeur de transpiration (axilliaire), on note deux choses :
- des odeurs agréables : notes musquée, fruitée, mielleuse, épicée et ambrée;
- des odeurs désagréables : notes aminée, aigre et fécale.

Selon l'invention, on ajoute un parfum capable à la fois d'exalter les odeurs agréables et atténuer, voire bloquer les odeurs désagréables.

L'invention a ainsi pour objet une composition cosmétique comprenant dans un véhicule à usage cosmétique :
(A) - un composé consistant en un inhibiteur enzymatique de la β-glucuronidase et de l'arylsulfatase;
(B) - un composé permettant d'élever le pH cutané au niveau des zones de transpiration à pH légèrement basique compris entre 7 et 8, de préférence entre 7 et7,5;
(C) - un composé diminuant la biodisponibilité de l'eau, ledit composé étant notamment choisi parmi les substances présentes dans la transpiration à l'état naturel ; et
   - un véhicule à usage cosmétique.

Selon un mode de réalisation préféré, la composition comprend :
(A) - de 0,02 à 1 % d'un composé consistant en un inhibiteur enzymatique de la β-glucuronidase et de l'arylsulfatase;
(B) - une quantité suffisante d'un composé permettant d'élever le pH cutané au niveau des zones de transpiration à pH légèrement basique, de préférence pH 7-7,5;
(C) - de 1 à 10 % d'un composé diminuant la biodisponibilité de l'eau, ledit composé étant notamment choisi parmi les substances présentes dans la transpiration à l'état naturel; et
   - un véhicule à usage cosmétique.

Avantageusement, la composition comprend en outre :
(D) - un composé apte à stabiliser le développement bactérien de la sueur, ledit composé étant notamment choisi parmi les substances présentes dans la sueur à l'état naturel; et/ou
(E) - un composé apte à piéger les acides gras à chaine courte volatils; et/ou
(F) - un système parfum apte à exalter les odeurs agréables de la transpiration et à inhiber les odeurs désagréables de la transpiration.

Avantageusement, la composition comprend :
- de 0,05 à 0,5 % du composé (D) ; et/ou
- de 0,05 à 5 % du composé (E) ; et/ou
- de 0,1 à 2 % du composé (F).

Avantageusement,
- le composé (A) est un sel minéral de zinc, de préférence le sulfate de zinc en solution aqueuse;
- le composé (B) est un aminoacide basique notamment un aminoacide basique présent dans la sueur, la triéthanolamine ou un autre tampon tels les tampons commercialisés par SIGMA sous les dénominations TAPSO^{R} ou POPSO^{R}; correspondant respectivement à l'acide 3-[N-tris-(hydroxyméthyl)méthylamino]-2-hydroxy-propane sulfonique et à l'acide pipérazine-N,N'-bis (2-hydroxy-propane sulfonique);
- le composé (C) est de préférence le lactate de sodium;
- le composé (D) est de préférence l'acide paraaminobenzoïque; ce composé renforce l'action du précédent;
- le composé (E) est de préférence un oxyde de zinc ou de magnésium; pour les compositions aérosols, il conviendra de remplacer les oxydes de zinc ou de magnésium du fait de leur insolubilité dans l'eau, l'alcool ou en milieu hydroglycolique par un sel de zinc ou de magnésium, notamment un sel organique, par exemple le ricinoléate de zinc.
- le parfum comprend avantageusement :
   . un composant capable d'exalter les odeurs agréables naturellement présentes dans la transpiration,
   . un composant capable de masquer les odeurs désagréables naturellement présentes dans la transpiration;
   . éventuellement un composant ayant une activité antimicrobienne et une activité intrinsèque de parfum : thymol, vétivérol par exemple.

On utilise de façon préférée le produit commercialisé par la société BUSH, BOAKE et ALLEN sous la dénomination VEILEX apte à remplir les deux premières fonctions ci-dessus mentionnées.

A cet effet, il conviendra de vérifier la compatibilité du parfum, le cas échéant, avec le ricinoléate de zinc qui est un absorbeur d'odeurs.

Une composition préférée selon l'invention comprend :
(A) - de 0,02 à 1% d'un sel minéral de zinc.
(B) - une quantité suffisante pour stabiliser le pH de la composition d'une substance tampon choisie notamment parmi la triéthanolamine, le TAPSO^{R} et le POPSO^{R};
(C) - de 1 à 10% de lactate de sodium;
(D) - de 0,05 à 0,5% d'acide paraaminobenzoïque;
(E) - de 0,05 à 5% d'un composé choisi parmi l'oxyde de zinc, l'oxyde de magnésium et le ricinoléate de zinc
(F) - de 0,1 à 2% d'un système parfum comprenant un agent actif odorifère à activité antimicrobienne, choisi parmi le thymol ou le vétiverol, et du VEILEX^{R};
(G) - un véhicule à usage cosmétique.

L'excipient dépendra de la présentation du produit :
. aqueux ou hydroalcoolique pour un conditionnement à bille (roll-on)
. hydroalcoolique pour un vaporisateur aérosol
. hydroglycolique (gel) pour un bâton (stick).

L'invention a également pour objet l'utilisation d'un sel minéral de zinc, notamment le sulfate de zinc pour inhiber la formation d'odeurs corporelles désagréables liées au processus de transpiration, éventuellement conjointement à un ou plusieurs des ingrédients (A) à (F) tels que définis précédemment.

Dans les exemples qui suivent on distingue la partie "ACTIF" de l'excipient. Les quantités sont données en poids pour 100 parties.

### Exemple 1 :

### Stick déodorant

| | | |
|---|---|---|
| | (sulfate de zinc | 0,8 |
| | (lactate de sodium | 3 |
| actif | (acide paraaminobenzoïque | 0,3 |
| | (oxyde de zinc | 2 |
| | (parfum | 1 |
| | dipropylène glycol | 55 |
| | propylène glycol | 10 |
| | glycérine | 10 |
| | stéarate de sodium | 9 |
| | sel tétrasodique de l'éthylènediamine | 0,04 |
| | eau qsp | 100 |

Remarque : le pH de la formule est légèrement basique en raison de la présence du stéarate de sodium qui joue également le rôle d'excipient.

### Exemple 2 :

### Déodorant en aérosol

| | | |
|---|---|---|
| | (sulfate de zinc | 1 |
| | (TEA qsp pH 7,5 | |
| actif | (lactate de sodium | 2 |
| | (acide paraaminobenzoïque | 0,2 |
| | (ricinoléate de zinc | 3 |
| | (parfum | 1,5 |
| | alcool à 96° | 80 |
| | eau qsp | 100 |
| gaz propulseur : air comprimé, pression: 1,05 | | 10⁶ Pa |

### Exemple 3 :

### Déodorant à bille

| | | |
|---|---|---|
| | (sulfate de zinc | 0,5 |
| | (TEA qsp pH 7,5 | |
| actif | (lactate de sodium | 2 |
| | (acide paraaminobenzoïque | 0,3 |
| | (oxyde de magnésium | 3 |
| | (parfum | 0,4 |
| | ricin hydrogéné 400E | 0,4 |
| | alcool à 96° | 30 |
| | propylène glycol | 5 |
| | glycérine | 2 |
| | hydroxyéthyl-cellulose | 0,4 |
| | eau qsp | 100 |

## Revendications

1. Composition cosmétique comprenant :
(A) - un composé consistant en un inhibiteur enzymatique de la β-glucuronidase et de l'arylsulfatase;
(B) - un composé permettant d'élever le pH cutané au niveau de zones de transpiration à pH compris entre 7 et 8, de préférence entre 7 et 7,5;
(C) - un composé diminuant la biodisponibilité de l'eau, ledit composé étant notamment choisi parmi les substances présentes dans la transpiration à l'état naturel; et
- un véhicule à usage cosmétique.

2. Composition selon la revendication 1, caractérisée en ce qu'elle comprend :
(A) - de 0,02 à 1 % d'un composé consistant en un inhibiteur enzymatique de la β-glucuronidase et de l'arylsulfatase;
(B) - une quantité suffisante d'un composé permettant d'élever le pH cutané au niveau des zones de transpiration à pH compris entre 7 et 8, de préférence entre 7 et 7,5;
(C) - de 1 à 10 % d'un composé diminuant la biodisponibilité de l'eau, ledit composé étant notamment choisi parmi les substances présentes dans la transpiration à l'état naturel, et
- un véhicule à usage cosmétique.

3. Composition cosmétique selon la revendication 1, comprenant en outre :
(D) - un composé apte à stabiliser le développement bactérien de la sueur, ledit composé étant notamment chcisi parmi les substances présentes dans la sueur à l'état naturel.

4. Composition selon la revendication 3 caractérisée en ce qu'elle comprend :
(D) - de 0,05 à 0,5 % d'un composé apte à stabiliser le développement bactérien de la sueur, ledit composé étant notamment choisi parmi les substances présentes dans la sueur à l'état naturel.

5. Composition cosmétique selon la revendication 1 comprenant en outre :
(E) - un composé apte à piéger les acides gras à chaine courte volatils.

6. Composition selon la revendication 5, caractérisée en ce qu'elle comprend :
(E) - de 0,05 à 5 % d'un composé apte à piéger les acides gras à chaine courte volatils.

7. Composition cosmétique selon la revendication 1 comprenant en outre :
(F) - un parfum apte à exalter les odeurs agréables de la transpiration et à inhiber les odeurs désagréables de la transpiration.

8. Composition selon la revendication 7, caractérisée en ce qu'elle comprend :
(F) - de 0,1 à 2 % d'un parfum apte à exalter les odeurs agréables de la transpiration et à inhiber les odeurs désagréables de la transpiration.

9. Composition selon la revendication 1, caractérisée en ce que le composé (A) est un sel minéral de zinc, notamment de sulfate de zinc.

10. Composition selon la revendication 1, caractérisée en ce que le composé (C) est le lactate de sodium.

11. Composition selon l'une quelconque des revendications 3 à 10, caractérisée en ce que le composé (D) est l'acide paraaminobenzoïque.

12. Composition selon l'une quelconque des revendications 5 à 11, caractérisée en ce que le composé (E) est un oxyde ou un sel organique de zinc ou de magnésium.

13. Composition selon l'une quelconque des revendications 7 à 12, caractérisée en ce que le composé (F) est un système parfum comprenant au moins un composant apte à exalter les odeurs agréables de la transpiration et au moins un composant apte à inhiber les odeurs désagréables de la transpiration.

14. Composition selon la revendication 1, comprenant :
(A) - de 0,02 à 1% d'un sel minéral de zinc;
(B) - une quantité suffisante pour stabiliser le pH de la composition d'une substance tampon choisie notamment parmi la triéthanolamine, le TAPSO^{R} et le POPSO^{R};
(C) - de 1 à 10% de lactate de sodium;
(D) - de 0,05 à 0,5% d'acide paraaminobenzoïque;
(E) - de 0,05 à 5% d'un composé choisi parmi l'oxyde de zinc, l'oxyde de magnésium et le ricinoléate de zinc;
(F) - de 0,1 à 2% d'un système parfum comprenant éventuellement un agent actif odorifère à activité antimicrobienne, choisi parmi le thymol ou le vétiverol, et du VEILEX^{R}; et
(G) - un véhicule à usage cosmétique.

15. Composition selon l'une quelconque des revendications précédentes, caractérisée en ce qu'elle est sous forme d'un bâton solide,d'un aérosol ou d'un gel.

16. Procédé pour inhiber la formation d'odeurs corporelles désagréables dues au processus de transpiration consistant à appliquer sur la peau à l'emplacement des zones d'émission de transpiration :
(A) - un composé consistant en un inhibiteur enzymatique de la β-glucuronidase et de l'arylsulfatase;
(B) - un composé permettant d'élever le pH cutané au niveau des zones de transpiration à pH compris entre 7 et 8, de préférence entre 7 et 7,5;
(C) - un composé diminuant la biodisponibilité de l'eau, ledit composé étant notamment choisi parmi les substances présentes dans la transpiration à l'état naturel.

17. Procédé selon la revendication 16, caractérisé en ce qu'il comprend en outre l'application :
(D) - d'un composé apte à stabiliser le développement bactérien de la sueur, ledit composé étant notamment choisi parmi les substances présentes dans la sueur à l'état naturel.

18. Procédé selon la revendication 16, caractérisé en ce qu'il comprend en outre l'application:
(E) - d'un composé apte à piéger les acides gras à chaine courte volatils.

19. Procédé selon l'une quelconque des revendications 16 à 18, caractérisé en ce qu'il comprend en outre l'application:
(F) - d'un parfum apte à exalter les odeurs agréables de la transpiration et à inhiber les odeurs désagréables de la transpiration.

20. Utilisation d'un sel minéral de zinc, pour inhiber la formation d'odeurs corporelles désagréables liées au processus de transpiration.

21. Utilisation selon la revendication 20, caractérisée en ce que le sel minéral de zinc est le sulfate de zinc.

22. Utilisation selon la revendication 20 ou la revendication 21, caractérisée en ce que le sel minéral de zinc est utilisé conjointement à un ou plusieurs des ingrédients (A) à (F) définis aux revendications 1 à 8.
